# EUROPÄISCHE PATENTSCHRIFT

(11) **EP 0 668 053 B1**
(45) Veröffentlichungstag und Bekanntmachung des Hinweises auf die Patenterteilung: **29.08.2001**
(21) Anmeldenummer: 94102522.3
(22) Anmeldetag: 19.02.1994
(51) Int. Cl.: A61B 10/00

(54) **Medizintechnisches Schneidegerät mit Griff und ebener, zirkulärer Schneide zur Entnahme von Festgewebe**
Medico-technical cutting device with planar, circular cutting edge for solid tissue sampling
Instrument coupant medico-technique avec poignée et tranchant circulaire et plan, pour prélever des tissus rigides

(43) Veröffentlichungstag der Anmeldung: 23.08.1995
(73) Patentinhaber: Voigt, Holger, Dr. med., D-24568 Kaltenkirchen/Holstein (DE)
(72) Erfinder: Voigt, Holger, Dr. med., D-24568 Kaltenkirchen/Holstein (DE)

(56) Entgegenhaltungen:
- FR-A- 2 238 464
- FR-A- 2 450 597
- US-A- 3 515 128
- US-A- 4 043 322
- US-A- 5 259 391

## Beschreibung

Die Erfindung betrifft ein medizintechnisches Schneidegerät mit Griff und ebener, zirkulärer Schneide zur Entnahme von Festgewebe.
Die Entfernung von Festgewebe zur diagnostischen Verarbeitung oder zur Therapie erfolgt in der Medizin überwiegend durch Anwendung schneidender Verfahrenstechniken unter Zuhilfenahme spezieller, als Skalpell bezeichneter Schneidemesser. Bei allen medizinisch verwendeten Schneidemessern dient ein spezieller Schliff der Schneide dem Zweck, einen glatten, scharfrandigen Schnitt zu gewährleisten, der erforderlich ist, um möglichst optimale Voraussetzungen zur Wundheilung zu schaffen. Um ein weitgehend ermüdungsfreies Arbeiten bei für das Schneiden günstigen Hebelverhältnissen zu gewährleisten, ist die Formgebung des die Schneide tragenden und bei der Ausführung der Schneidefunktion von Fingern und Hohlhand der Arbeitshand fixierten und demzufolge zumindest fingerlangen Griffes der Aufgabenstellung entsprechend angepaßt.
Handelt es sich hinsichtlich des Einsatzgebietes um größenordnungsmäßig kleine Schneidevorgänge im Bereich weniger Millimeter Durchmesser an einsehbaren und leicht zugänglichen Körperoberflächen können alternativ auch Schneidegeräte verwendet werden, an deren Griff endständig eine ebene, zirkuläre Schneide angebracht ist. Dieses hat den Vorteil, den Schneidevorgang in seiner technischen Ausführung in der Weise zu vereinfachen, daß anstelle eines Gewebeeinschnittes mit anschließender Gewebepräparation zur endgültigen Abtrennung des Gewebestückes durch senkrechtes Aufdrücken einer an einem Griff befestigten ebenen, zirkulären Schneide und anschließender Drehung der auf eine Gewebeoberfläche aufgesetzten Schneide der gesamte Schneidevorgang abgekürzt wird. Der herausgeschnittene Gewebezylinder verbleibt im hohlen Griff und ist bei Entfernen des Gerätes von der Gewebeoberfläche von darunter liegenden Gewebsstrukturen leicht durch Scherenschlag abtrennbar (Stegman SJ, Tromovitch TA, Glogau RG: Basis of Dermatologic Surgery. Chicago, Year Book Medical Publishers, 1982: 1-51).
Wenn auch die Gewebeentnahme durch Verwendung derartiger Schneidegeräte im Gegensatz zur herkömmlichen Schneidetechnik mit Skalpellmessern vereinfacht durchführbar ist, ist es bislang nicht möglich gewesen, Schneidegeräte mit ebener, zirkulärer Schneide zur Entnahme von Festgewebe im Bereich von Körperregionen einzusetzen, in denen eingeschränkte oder fehlende Sicht- und Manövrierverhältnisse vorliegen. Derartige Körperregionen stellen z.B. die von außen nicht direkt einsehbaren Anteile von Haut und Schleimhaut im Bereich der Mundhöhle, des Gehörganges, der Enddarm- und Genitalregion dar sowie Bereiche mit enganeinanderliegenden Gewebsstrukturen, z.B. Körperfalten im Achsel-, Brust-, Bauch- und Gesäßbereich sowie der Bereich der Zehenzwischenräume. In diesen Bereichen läßt sich ein Schneidegerät mit ebener, zirkulärer Schneide nicht exakt zielbezogen lokalisieren und ansetzen, da einerseits die Einsicht in das Gebiet eingeschränkt oder fehlend ist, andererseits zur Durchführung des Schneidevorganges eine zur Gewebeoberfläche senkrechte, aufrechte Positionierung eines eine Schneide tragenden, in der Arbeitshand zu fixierenden Griffes erforderlich ist, was infolge eingeschränkten Manövrierraumes mißlingt. Der Versuch, über flachwinkliges oder gar tangentiales Ansetzen der zirkulären Schneide Gewebe zu entnehmen, schlägt fehl, da durch ungleichmäßige Verteilung des auf die Schneide wirkenden Druckes bei der erfolgenden Drehbewegung kein gleichmäßiges Durchtrennen des Gewebes erzielt wird und auf diese Weise ungleiche Schneidetiefe sowie damit verbunden gewebsstauchende und gewebsausziehende Effekte resultieren, die ihrerseits die nachfolgende Wundheilung negativ beeinflussen.
Die Aufgabe, auch in sicht- und manövrierraumbegrenzten Körperregionen die Entnahme von Festgewebe durch Verwendung eines Schneidegerätes mit ebener, zirkulärer Schneide durchführen zu können, wird erfindungsgemäß entsprechend Anspruch 1 und 2 in der Weise gelöst, daß ein eine ebene, zirkuläre Schneide 2 aufweisender, innen hohler Griff 1 eine zum Lot einer durch die Umfangslinie der Schneide 2 gebildeten Schneidenebene 6 rechtwinklige Abbiegung 3 von einem Schneidenansatzteil 4 aufweist, in deren äußeren Scheitelpunkt sich eine Öffnung 5 befindet, die in gerader Linie gegenüber der Schneidenebene 6 angeordnet ist. Durch Verwendung eines derartig konstruierten Schneidegerätes ist es bei der Ausführung des Schneidevorganges möglich, in Regionen mit eingeschränkten Sicht- und Manövrierverhältnissen eine ebene, zirkuläre Schneide 2 senkrecht auf einer Gewebeoberfläche anzusetzen, wobei die sich im äußeren Scheitelpunkt des Griffes 1 befindliche Öffnung 5 eine Längenbegrenzung des ausgeschnittenen festen, jedoch verformbaren Gewebezylinders bewirkt. Durch Druck des Zeigefingers der das Gerät einführenden und haltenden Hand auf die rückwärtige Öffnung 5 des Griffes 1, die gleichzeitig auch als taktile Führungshilfe zur Lokalisation und Plazierung der Schneide 2 dient, wird die Schneide 2 senkrecht zur Oberfläche in das Gewebe eingeführt, worauf im Anschluß nach Erreichen der gewünschten Gewebetiefe bei unveränderter Position von Hand und Finger allein durch seitliche Drehung des rechtwinklig abgebogenen Griffes 1 ein Gewebezylinder herausgeschnitten und in den Hohlraum des Schneidenansatzteiles 4 hineingeschoben wird. Dabei wird die Länge des ausgeschnittenen Gewebezylinders durch die darüberliegende Öffnung 5 sowie die rechtwinklige Abbiegung 3 im Scheitelbereich des Griffes 1 begrenzt. Nach Entfernung des Schneidegerätes aus dem Manövriergebiet wird der im Hohlraum des Schneidenansatzteiles 4 befindliche Gewebezylinder durch die im Griff 1 befindliche Öffnung 5 in ein Auffanggefäß ausgestoßen. Der Raumbedarf für das Einbringen, Schneiden und Herausführen des Gerätes wird insgesamt allein durch die Höhe von Griff 1, Schneidenansatzteil 4 und aufliegendem Zeigefinger bestimmt.

Die Erfindung kann in Bereichen der Medizin, Biologie und Pathologie zur gewebsschonenden schneidenden Entnahme von Festgewebe eingesetzt werden, insbesondere an Lokalisationen, die von außen eingeschränkt oder nicht einsehbar sind und eine Begrenzung des Manövrierraumes bedingen.

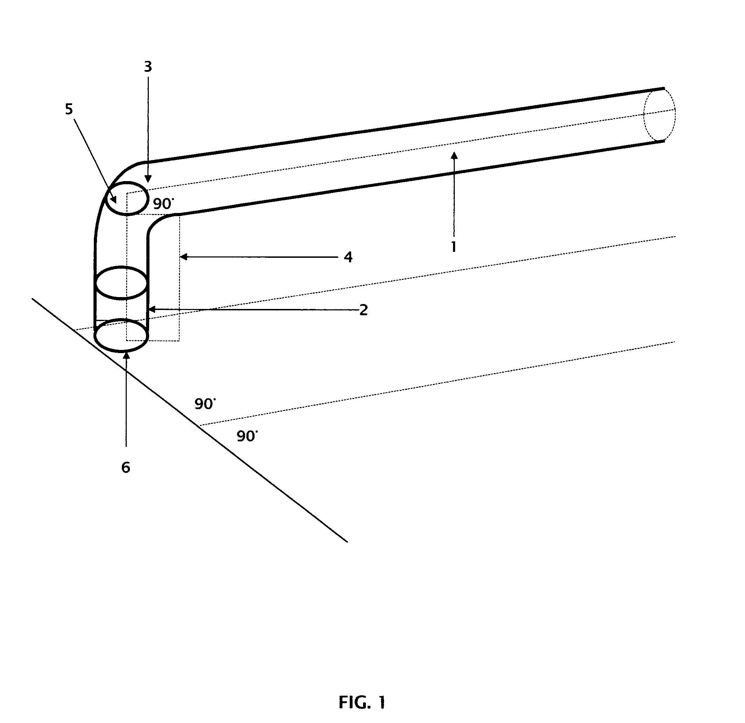

Das Gerät ist schematisch in Abbildung 1 dargestellt.

Ein im Innenraum hohler Griff 1, der bei der Ausführung der Funktion von Fingern und Hohlhand der Arbeitshand fixiert wird, weist endständig eine ebene, zur Achse des Griffes 1 senkrecht stehende, zirkuläre Schneide 2 auf. Der Griff 1 weist eine zum Lot einer durch die Umfangslinie der Schneide 2 gebildeten Schneidenebene 6 rechtwinklige Abbiegung 3 eines Schneidenansatzteiles 4 auf. In der Oberfläche des Griffes 1 befindet sich in Höhe des Scheitelpunkts der rechtwinkligen Abbiegung 3 vom Schneidenansatzteil 4 eine Öffnung 5, die in gerader Linie gegenüber der durch die Umfangslinie der endständigen Schneide 2 gebildeten Ebene 6 angeordnet ist.

## Patentansprüche

1. Medizintechnisches Schneidegerät zur Entnahme von Festgewebe mit einem innen hohlen Griff (1) und einer an einem Ende des Griffs (1) und senkrecht zur Achse des Griffs (1) angeordneten, ebenen und zirkulären Schneide (2), dadurch gekennzeichnet, daß der Griff (1) eine zum Lot der Schneidenebene (6) rechtwinklige Abbiegung (3) von einem Schneidenansatzteil (4) aufweist, in deren äußeren Scheitelpunkt sich eine Öffnung (5) befindet, die in gerader Linie gegenüber der durch die Umfangslinie der endständigen Schneide (2) gebildeten Ebene (6) angeordnet ist.

2. Medizintechnisches Schneidegerät nach Anspruch 1, wobei der Durchmesser der im Griff (1) befindlichen Öffnung (5) geringer ist als der der zirkulären Schneide (2).

## Claims

1. Medico-technical cutting device for solid tissue sampling comprising an inner hollow handle (1) and a planar circular blade (2) positioned at one end of the handle (1) and at a right angle to the axis of the handle (1), characterised in that the handle (1) has a right-angled bend (3) of a blade extension part (4) perpendicular to the cutting plane (6), having an opening (5) at its external apex, which is being arranged in straight line opposite to the plane (6) formed by the circumference of the terminal blade (2).

2. Medico-technical cutting device according to claim 1, wherein the diameter of the opening (5) of the handle (1) is being smaller than that of the circular blade (2).

## Revendications

1. Instrument de dissection médico-technique pour prélever des tissus rigides, comprenant un manche (1) à volume intérieur creux et un tranchant (2) plan et circulaire disposé à une extrémité du manche (1) perpendiculairement à l'axe du manche (1), caractérisé en ce que le manche (1) présente une courbure (3) par une prolongation (4) du tranchant, courbure à angle droit par rapport à la verticale du plan de coupe (6) et dans l'extrémité apicale extérieure de laquelle se trouve une ouverture (5), disposée en ligne droite par rapport au plan de coupe (6) formé par la ligne circonférentielle du tranchant (2) terminal.

2. Instrument de dissection médico-technique selon la revendication 1, dans lequel le diamètre de l'ouverture (5) ménagée dans le manche (1) est inférieur à celui du tranchant circulaire (2).
